# EUROPEAN PATENT APPLICATION

(11) **EP 4 116 728 A1**
(43) Date of publication of application: **11.01.2023**
(21) Application number: 21764038.2
(22) Date of filing: 05.03.2021
(51) Int. Cl.: G01R 33/485, G01N 24/00, G01N 24/08, A61B 5/055

(54) **NUCLEAR MAGNETIC RESONANCE MEASUREMENT METHOD AND NUCLEAR MAGNETIC RESONANCE APPARATUS**

(30) Priority: 06.03.2020 JP 2020039397
(71) Applicant: National University Corporation Kochi University, Kochi-shi, Kochi 780-8520 (JP); Spectro Decypher, Inc., Minato-ku Tokyo 107-0062 (JP)
(72) Inventor: Tsuda Masashi, Kochi-shi, Kochi 780-8520 (JP); TSUDA Masayuki, Kochi-shi, Kochi 780-8520 (JP); NAKAYAMA Noboru, Tokyo 107-0062 (JP); NAKAOKA Shigeru, Tokyo 107-0062 (JP)
(74) Representative: Beyer, Andreas
(86) International application number: PCT/JP2021/008841
(87) International publication number: WO 2021/177465

(57) **Abstract**

A subject S to which ¹⁷O gas has been administered is placed within a fixed uniform static magnetic field of an NMR apparatus 1. The subject is irradiated, through proton coupling, with an excitation pulse produced using a pulse sequence having a short cycle time of 20.4 msec or less, preferably 10.4 msec or less, and more preferably 5.6 msec or less. An NMR signal generated due to ¹⁷O nuclei of ¹⁷O water produced within the subject by oxygen metabolism of the ¹⁷O gas being excited by irradiation with the excitation pulse is detected with high sensitivity and is processed in accordance with a prescribed imaging sequence in which an MRS sequence is used.

## Description

### Technical Field

The present invention relates to a nuclear magnetic resonance measurement method and a nuclear magnetic resonance apparatus in which ¹⁷O in a subject is non-invasively detected and measured by using a nuclear magnetic resonance (NMR) phenomenon.

### Background Art

In the prior art, inspection methods such as magnetic resonance imaging (MRI) and computed tomography (CT) capable of non-invasively viewing a structure in a brain have been used in order to detect and diagnose diseases such as cerebral hemorrhage, cerebral infarction, and brain tumor. However, with MRI and CT, it is not possible to accurately view which brain cells are active or inactive, and, it is not possible to diagnose parts having a poor brain function, improvement effects, and the like for brain diseases such as Alzheimer-type dementia.

In recent years, research has been conducted to be used to diagnose various diseases by administering ¹⁷O nucleus-labeled water (H₂¹⁷O, hereinafter referred to as "¹⁷O water") to a living body and non-invasively observing ¹⁷O water stored in the living body by means of MRI to detect a blood flow rate and oxygen metabolism in the living body. For example, a method of operating an MRI apparatus that enables a cerebral blood flow inspection with high time resolution by performing imaging using ¹⁷O water has been proposed (see, for example, PTL1). In this method, H nucleus (proton) of ¹⁷O water is selected as an imaging target nucleus, and T2 relaxation of the selected H nucleus is measured and imaged by the spin echo method. Thus, even when the intravascular concentration of ¹⁷O water is low, it is possible to perform imaging at the resonance frequency of protons by means of the MRI apparatus that is widely used. In addition, it is possible to sufficiently capture a signal change.

In general, it has been reported that, in NMR measurements performed by exciting a nucleus having a quadrupole bipolar moment, such as a ¹⁷O nucleus, a half-value width of an NMR signal changes, due to differences in chemical bonds and ligands, that is, depending on the states of electrons around a ¹⁷O molecule, which affects and shortens the relaxation time of ¹⁷O nuclei (see, for example, NPTL1 and NPTL 2). According to NPTL1 and NPTL2, proton atoms of a water molecule are bonded not with a covalent bond but with an ionic bond and slowly exchanges with the surrounding proton atoms. Thus, the relaxation time of the ¹⁷O nucleus is extremely short.

Recently, in order to improve the sensitivity and resolution of measurements, a method of directly performing NMR-measurement of a ¹⁷O atom by using MRS (magnetic resonance spectroscopy and 2D CSI (chemical shift imaging) and applying the fact that the relaxation time of ¹⁷O nuclei is short has been reported (see, for example, NPTL 3). According to this report, the optimum cycle time (TR) of a pulse sequence for ¹⁷O-NMR measurement was TR = 15 msec.

### Citation List

### Patent Literature

PTL1: Japanese Unexamined Patent Publication No. 2013-255586

### Non-Patent Literatures

NPTL1: J. C. Hindman et.al, "Relaxation Processes in Water. The Spin-Lattice Relaxation of the Deuteron in D2O and Oxygen-17 in H217O", J. Chem. Phys., 54(1971), 621
NPTL2: J. C. Hindman, "Relaxation processes in water: Viscosity, self-diffusion, and spin-lattice relaxation. A kinetic model", J. Chem. Phys., 60(1974), 4488
NPTL3: X.H. Zhu et.al, "17O Relaxation Time and NMR Sensitivity of Cerebral Water and Their Field Dependence", 2001 Magn. Reson. Med., 45(2001), 543-549

### Summary of the Invention

### Technical Problem

Since MRI is an image in which the relaxation of the H nucleus is emphasized, the detected peak intensity of the H nucleus of the ¹⁷O water can be imaged. However, there is a problem that it is not possible to accurately reflect the state of a part at which the ¹⁷O water is present. Since the conventional method disclosed in PTL1 does not directly measure the ¹⁷O nucleus, the sensitivity and resolution in the measurement are limited. Therefore, in imaging in the brain, the T2 relaxation time differs depending on the relative relationship with the ¹⁷O concentration, for example, for each of parts such as cerebrospinal fluid, gray matter, and white matter. Thus, there is a concern that the sensitivity of ¹⁷O obtained as a result is ambiguous.

Further, in the method disclosed in PTL1, it is possible to detect a sufficient signal change by intravenously administering high-concentration ¹⁷O water to the subject, and to obtain maps of the cerebral blood flow rate and the cerebral blood volume with high time resolution. According to the same document, the concentration of ¹⁷O water is preferably as high as 10% by weight or more, for example. However, such high-concentration ¹⁷O water has a problem that the natural abundance ratio of ¹⁷O nuclei is as low as 0.038%, and thus high-concentration ¹⁷O water is expensive and cannot be easily obtained.

On the other hand, in an NMR apparatus with high resolution for ¹³C nuclei, a 1H-detected 1H-¹³C shift-correlated two-dimensional NMR (1H-detected Multiple Quantum Coherence spectrum (HMQC)) method, which is called an inverse measurement method, and a 1H-detected 1H-¹³C long range shift-correlated two-dimensional NMR (1H-detected Multi-Bond heteronuclear multiple quantum Coherence spectrum (HMBC)) method are conventionally used. In these methods, a measurement is performed by exciting ¹³C nuclei and transferring energy to the H nuclei attached to the ¹³C nuclei. The inventors of the present application applied the NMR methods and tested a method of administering ¹⁷O gas to a subject which is a living body such as a human or an animal, exciting ¹⁷O nuclei of ¹⁷O water produced from oxygen metabolism of the ¹⁷O gas, and detecting H nuclei to which energy was transferred. However, it was not possible to detect the H nuclei.

As a result of various studies, the inventors of the present application have found that the cause is that the relaxation time of ¹⁷O nucleus of water is very short, which is more than two orders of magnitude shorter than the relaxation time of the ¹³C nucleus of a general organic compound. Then, when the cycle time of the pulse sequence in the NMR measurement was set in accordance with such a short relaxation time, it came to the conclusion that there was no time to transfer the energy to the H nucleus.

The inventors of the present application considered that: ¹⁷O, which is an oxygen isotope that can be measured by NMR, is used as a label, a ¹⁷O gas is administered to a living body and then ¹⁷O-NMR is measured on a target affected part (for example, brain cells), thereby making it possible to visually observe the intensity, the half-value width, the integrated value, and the chemical shift of the detected NMR signal and to more accurately determine the state of ¹⁷O atoms in the living body (for example, activation of brain cells). Furthermore, the inventors of the present application considered that it is possible to perform the measurement with higher sensitivity than using the conventional MRI method, by exciting the ¹⁷O nucleus and measuring the NMR of ¹⁷O.

On basis of the above consideration, in the NMR measurement performed by exciting the ¹⁷O nucleus of ¹⁷O water, the inventors reduced the cycle time of the pulse sequence to be shorter than the cycle time for the general NMR measurement (for example, around 2s) performed by exciting the ¹³C nucleus described above, by two orders of magnitude or more, and then measured the ¹⁷O nucleus itself. Thus, the sensitivity was improved by 100 times or more. However, with this degree of sensitivity improvement, it was not possible to sufficiently detect ¹⁷O nuclei with a natural abundance ratio as low as 0.038%.

The present invention has been made based on such findings. An object of the present invention is to provide a nuclear magnetic resonance measurement method capable of detecting ¹⁷O present in a subject with high sensitivity by using an NMR phenomenon, in particular, a magnetic resonance spectroscopy method (MRS method) of obtaining, from detected signals, a spectrum in which the signal intensity is on the vertical axis and the frequency is on the horizontal axis.

Further, an object of the present invention is to make it possible to detect ¹⁷O present in a subject at a level as low as the natural abundance ratio, with high sensitivity.

### Solution to Problem

According to the present invention, a nuclear magnetic resonance measurement method includes steps of:
placing a subject in a predetermined uniform static magnetic field and irradiating the subject with an excitation pulse generated in accordance with a predetermined pulse sequence;
detecting an NMR signal generated by exciting a ¹⁷O nucleus of ¹⁷O water present in the subject at the natural abundance ratio with the excitation pulse; and
processing the detected NMR signal,
in which a cycle time of the predetermined pulse sequence of the excitation pulse is 20.4 msec or shorter.

In an embodiment, the cycle time of the predetermined pulse sequence is set to 10.4 msec or shorter. In another embodiment, the cycle time of the predetermined pulse sequence is set to 5.6 msec or shorter.

In an embodiment, the cycle time of the predetermined pulse sequence is in a range of 5.6 msec to 2.8 msec.

In another embodiment, the irradiation with the excitation pulse is performed under proton decoupling.

In an embodiment, the nuclear magnetic resonance measurement method further includes a step of administering a ¹⁷O gas to the subject, which is a living body, before placing the subject in the static magnetic field, and the NMR signal generated by exciting a ¹⁷O nucleus of ¹⁷O water produced in the living body by oxygen metabolism of the ¹⁷O gas with the excitation pulse is detected.

In another embodiment, the nuclear magnetic resonance measurement method further includes a step of applying a gradient magnetic field pulse to the static magnetic field, the gradient magnetic field pulse generated in accordance with a predetermined imaging sequence.

According to another aspect of the present invention, a nuclear magnetic resonance apparatus includes:
a static magnetic field generation unit generating a predetermined uniform static magnetic field in a space where a subject is placed;
a radio-frequency pulse generation unit generating an excitation pulse, which is an RF pulse for exciting a ¹⁷O nucleus present in the subject, in accordance with a predetermined pulse sequence;
an NMR probe irradiating the subject placed in the static magnetic field with the excitation pulse and detecting an NMR signal generated from the ¹⁷O nucleus of the subject excited by the excitation pulse; and
a control unit controlling operations of the static magnetic field generation unit, the radio-frequency pulse generation unit, and the NMR probe,
in which the control unit controls the radio-frequency pulse generation unit to generate the excitation pulse in accordance with the predetermined pulse sequence having a cycle time of 20.4 msec or shorter.

In an embodiment, the cycle time of the predetermined pulse sequence is set to 10.4 msec or shorter. In another embodiment, the cycle time of the predetermined pulse sequence is set to 5.6 msec or shorter.

In an embodiment, the cycle time of the predetermined pulse sequence is in a range of 5.6 msec to 2.8 msec.

In another embodiment, the radio-frequency pulse generation unit generates a decoupling pulse, which is an RF pulse for performing proton decoupling that eliminates an influence of scalar coupling between a ¹H nucleus and a ¹⁷O nucleus of ¹⁷O water, and the NMR probe irradiates the subject with the decoupling pulse.

In an embodiment, the nuclear magnetic resonance apparatus further includes a gradient magnetic field generation unit applying a gradient magnetic field pulse generated in accordance with a predetermined imaging sequence to the static magnetic field.

In this case, the control unit performs a process for imaging the NMR signal detected by the NMR probe.

### Advantageous Effect of the Invention

According to the method and apparatus of the present invention, the sensitivity of detecting a ¹⁷O nucleus of ¹⁷O water in a subject is significantly improved by performing irradiation with the excitation pulse in accordance with a pulse sequence having a cycle time that is much shorter than before. In addition to improvement of the sensitivity of one cycle time, it is possible to significantly increase the number of integrations as compared to a usual case, during the same measurement time as the cycle time of chemical shift imaging of normal ¹⁷O-NMR measurement, because the cycle time of the pulse sequence is very short. Thus, the detection sensitivity of a ¹⁷O nucleus is further improved. As a result, it is possible to measure ¹⁷O nucleus present in the subject at the same concentration as the natural abundance ratio, with very high sensitivity.

### Brief Description of Drawings

[FIG. 1] FIG. 1 is a block diagram showing a preferred configuration of an NMR apparatus according to the present invention.
[FIG. 2] FIG. 2 is a flowchart illustrating an NMR measurement method according to a preferred embodiment of the present invention.
[FIG. 3] FIG. 3 is a diagram showing a measurement pulse sequence used in the NMR apparatus of the present embodiment.
[FIG. 4] FIG. 4 is an NMR spectrum diagram showing a result of detecting a ¹⁷O nucleus of 1% ¹⁷O water by a conventional pulse sequence used in Comparative Example 1.
[FIG. 5] FIG. 5 is an NMR spectrum diagram showing a result of detecting a ¹⁷O nucleus of 1% ¹⁷O water by a pulse sequence used in Comparative Example 2 and having a cycle time shorter than that of Comparative Example 1.
[FIG. 6] FIG. 6 is an NMR spectrum diagram showing a result of detecting a ¹⁷O nucleus of 1% ¹⁷O water by a pulse sequence used in Comparative Example 3 and having a cycle time much shorter than that of Comparative Example 1.
[FIG. 7] FIG. 7 is a graph showing a comparison in signal intensity of a ¹⁷O nucleus detected in Example 2 between a case where proton decoupling is performed and a case where proton decoupling is not performed.
[FIG. 8] FIG. 8 is a diagram showing a result of an In-vivo experiment in Example 3, where FIG. 8(a) is an NMR spectrum diagram showing a result of detecting a ¹⁷O nucleus, FIG. 8(b) is an NMR spectrum diagram showing a result of detecting a ¹⁷O nucleus when proton decoupling is performed, and FIG. 8(c) is a 3D image of a mouse brain, which has been measured and processed by using MRS.
[FIG. 9] FIG. 9 is a graph showing a change in signal intensity of a ¹⁷O nucleus detected in Example 4.
[FIG. 10] FIG. 10 is an NMR spectrum diagram showing a result of detecting a ¹⁷O nucleus in a case of a cycle time TR = 20.4 msec in Example 5.
[FIG. 11] FIG. 11 is an NMR spectrum diagram showing a result of detecting a ¹⁷O nucleus in a case of a cycle time TR = 10.4 msec in Example 5.
[FIG. 12] FIG. 12 is an NMR spectrum diagram showing a result of detecting a ¹⁷O nucleus in a case of a cycle time TR = 5.6 msec in Example 5.
[FIG. 13] FIG. 13 is an NMR spectrum diagram showing a result of detecting a ¹⁷O nucleus in a case of a cycle time TR = 2.8 msec in Example 5.

### Description of Embodiments

With reference to the accompanying drawings, an NMR measurement method according to preferred embodiments of the present invention will be described in detail below. In the present embodiment, a case where a living body such as a human or an animal is used as a subject will be described. However, the present invention is not limited to this case, and can be similarly applied to any object such as water and an aqueous solution containing ¹⁷O as a subject.

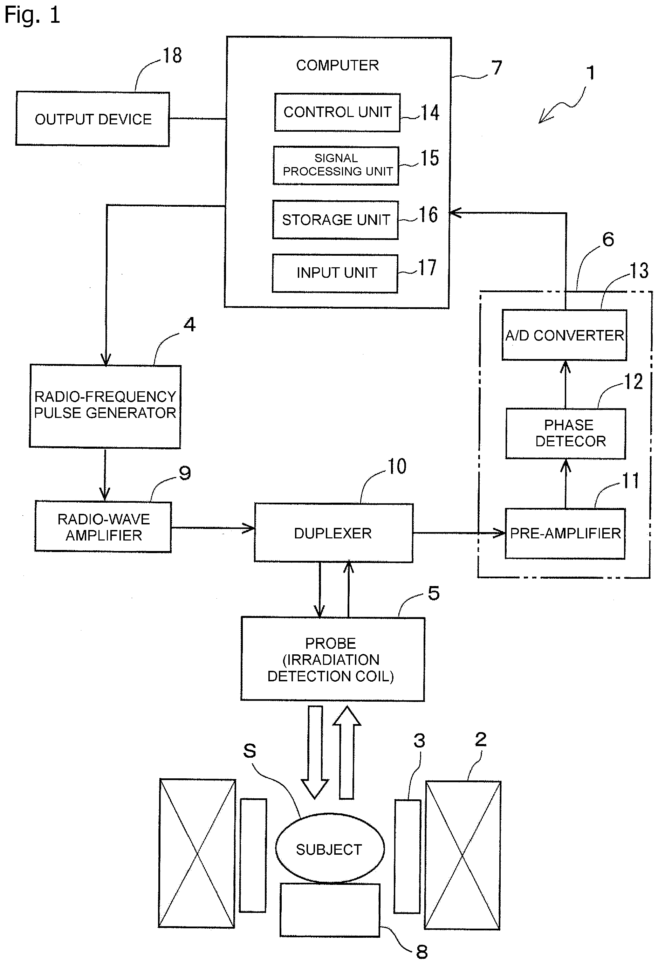

FIG. 1 schematically shows a preferred configuration of an NMR apparatus according to the present invention. An NMR apparatus 1 includes a static magnetic field generation unit 2, a gradient magnetic field generation unit 3, a radio-frequency pulse generator 4 which generates a radio-frequency pulse (hereinafter referred to as an RF pulse) as a radio wave, an NMR probe 5 which performs irradiation with the RF pulse and detection of an NMR signal, a signal receiving system 6 which amplifies and detects the NMR signal, and a computer 7. The NMR apparatus 1 is a multi-nuclear NMR apparatus capable of performing NMR measurements targeting atomic nuclei other than ¹H and ¹³C.

The static magnetic field generation unit 2 is configured by a superconducting magnet, a permanent magnet, an electromagnet, or the like, and generates a desired uniform static magnetic field in a space where a subject is placed. A table 8 for placing a subject S is provided in the space where the static magnetic field is generated. The static magnetic field generation unit 2 is configured by a cylindrical superconducting magnet as shown in FIG. 1. In another embodiment, the static magnetic field generation unit 2 can be configured by a split type superconducting magnet which generates a horizontal magnetic field.

The gradient magnetic field generation unit 3 is disposed inside the static magnetic field generation unit 2, and includes three independent sets of coils in order to generate a gradient magnetic field in the orthogonal three axis (X-axis, Y-axis, Z-axis) directions with certain strength. The gradient magnetic field generation unit 3 generates a gradient magnetic field in the X, Y, and Z directions in a pulsed manner. The gradient magnetic field is superimposed on the static magnetic field generated by the static magnetic field generation unit 2. In the gradient magnetic field, the magnetic field strength is spatially changed in a gradient manner.

The radio-frequency pulse generator 4 generates an RF pulse at a predetermined resonance frequency at which a measurement target nucleus of the subject S, that is, a ¹⁷O nucleus is excited. Specifically, the resonance frequency of the ¹⁷O nucleus in a magnetic field of 1.5 T is 8.658 MHz. A radio-wave amplifier 9 amplifies the RF pulse from the radio-frequency pulse generator 4 and transmits the amplified RF pulse to the NMR probe 5 via a duplexer 10.

The radio-frequency pulse generator 4 further generates a decoupling pulse, which is an RF pulse with which the ¹H nucleus is irradiated, in order to perform proton decoupling that eliminates the influence of scalar coupling between the ¹H nucleus and the ¹⁷O nucleus of ¹⁷O water. The decoupling pulse is generated with a ¹H nuclear signal (399.874 MHZ) of ¹⁷O water as a center frequency and a predetermined width of, for example, ±5,000 Hz. Then, the subject S is similarly irradiated with the decoupling pulse from the NMR probe 5.

The NMR probe 5 irradiates the subject S with an excitation pulse, which is an amplified RF pulse, via an irradiation detection coil inside the NMR probe 5. The irradiation detection coil of the NMR probe 5 is configured by a saddle-type probe coil when the static magnetic field generation unit 2 is configured by the cylindrical superconducting magnet of FIG. 1. When the static magnetic field generation unit 2 is configured by a split type superconducting magnet, the irradiation detection coil is configured by a solenoid type probe coil, corresponding to the shape of the split type superconducting magnet.

The irradiation detection coil in the NMR probe 5 detects an induced current generated by the atomic nuclear spin of the ¹⁷O nucleus of the subject S excited by the RF pulse and then outputs the detected induced current as an NMR signal (free induction decay signal: FID signal) to the duplexer 10. The duplexer 10 separates the NMR signal from the RF pulse and transmits the NMR signal to the signal receiving system 6.

The signal receiving system 6 is configured by a pre-amplifier 11, a phase detector 12, and an A/D converter 13. In the SIGNAL receiving system 6, the pre-amplifier 11 amplifies the NMR signal from the duplexer 10, and the phase detector 12, to which a reference signal is supplied from the radio-frequency pulse generator 4, detects the phase of the amplified NMR signal. The phase-detected signal is divided into signals in two orthogonal systems, and then the A/D converter 13 digitally converts the divided signals and transmits the digitally-converted signals to the computer 7.

The computer 7 includes a control unit 14, a signal processing unit 15, a storage unit 16, and an input unit 17, which are configured by a CPU(central processing unit), a RAM (random access memory), a ROM (read only memory), and the like. The computer 7 is connected to an external output device 18, for example, a display or a printer.

The control unit 14 controls the operations of the signal processing unit 15, the storage unit 16, the input unit 17 of the computer 7, and the external output device 16, in addition to various devices such as a radio-frequency pulse generator 4, the static magnetic field generation unit 2, and the gradient magnetic field generation unit 3 that constitute the NMR apparatus 1. In particular, the control unit 14 controls the radio-frequency pulse generator 4, the gradient magnetic field generation unit 3, and the signal receiving system 6 to apply an RF pulse and a gradient magnetic field pulse in accordance with a predetermined imaging sequence and process the detected NMR signal.

The signal processing unit 15 performs signal processing, which is necessary for obtaining a desired NMR spectrum, on the digitally-converted NMR signal supplied from the signal receiving system 6 under the control of the control unit 14. The storage unit 16 stores the digitally-converted NMR signal and an NMR spectrum obtained by the signal processing of the signal processing unit 15, under the control of the control unit 14.

The signal processing performed by the signal processing unit 15 includes a process of Fourier-transforming the digitally-converted NMR signal, an integration process of integrating the Fourier-transformed NMR signal, that is, the FID signal, and an imaging process. In the imaging process, the position information in the three axis directions of X, Y and Z is encoded into the NMR signal by the pulse of the gradient magnetic field applied by the gradient magnetic field generation unit 3. The signal processing unit 15 further performs phase correction of an NMR spectrum signal, if necessary.

The input unit 17 is configured so that an operator can input necessary information such as control information of the NMR apparatus 1 and others via an external input device such as a keyboard or a mouse connected to the computer 7. The signal processing unit 15 creates measurement pulse sequence for determining application timings of the RF pulse and the gradient magnetic field pulse, based on the information input by the operator via the input unit 17. As the pulse sequence, a spuls (single pulse sequence) sequence provided as the standard in the NMR apparatus 1 or a pulse sequence obtained by improving the single pulse sequence in accordance with a desired measurement can be used. The imaging sequence for processing the received NMR signal is determined by the above pulse sequence and parameters such as the applied intensity of the RF pulse and the gradient magnetic field pulse. The signal processing unit 15 calculates the parameters based on imaging conditions input by the operator via the input unit 17.

In the above embodiment, the control unit 14, the signal processing unit 15, the storage unit 16, and the input unit 17 constitute one computer 7. In another embodiment, the signal processing unit 15 and the storage unit 16 can be divided and be configured by separate computers.

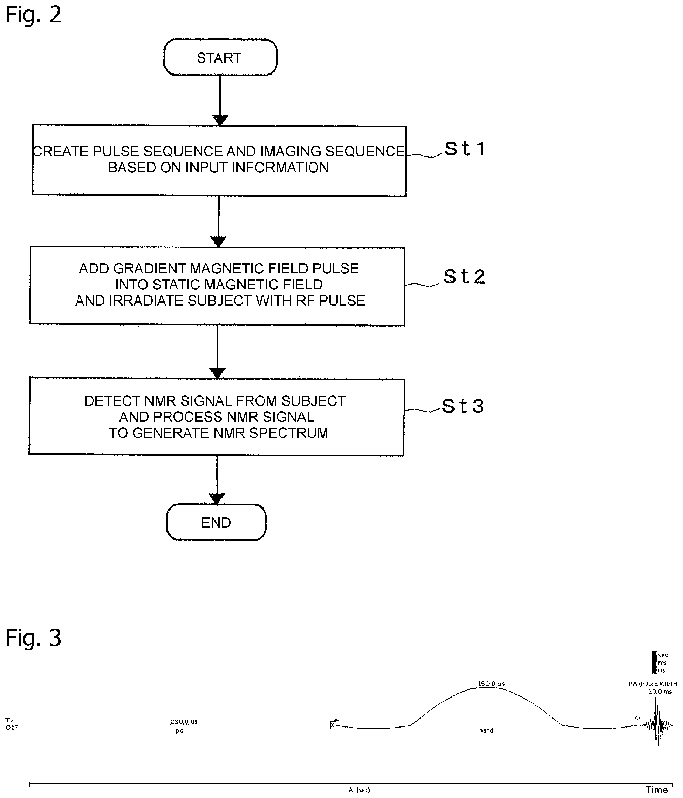

The NMR measurement method according to the present embodiment, which is performed in the NMR apparatus 1 in FIG. 1, will be described in detail with reference to the flowchart shown in FIG. 2. First, the signal processing unit 15 creates a measurement pulse sequence and an imaging sequence based on the above information input by the operator via the input unit 17 (Step St1).

In the present embodiment, the cycle time of the pulse sequence is a time obtained in a manner that an acquisition time (acq. time), that is, a measurement time from the excitation of the nuclear spin to the ¹⁷O nucleus by the irradiation with the RF pulse to the acquisition of the NMR signal generated by absorbing energy of the RF pulse absorption is added to a delay time until irradiation with an RF pulse is performed again for the next measurement. According to the present invention, the acquisition time can be set to a very short time, for example, 10 msec or less, in consideration of a very short relaxation time of ¹⁷O which is a detection atomic nucleus.

Similarly, the delay time is also very short and can be set to, for example, 10 msec. Thus, since the cycle time can be set to be significantly shorter than the normal cycle time, the measurement can be repeatedly performed a larger number of times within a shorter time than usual, and the integration efficiency is improved.

The subject S is administered with a ¹⁷O gas in advance, and is then placed on the table 8 of the NMR apparatus 1 a after a predetermined time has elapsed. The predetermined time is the time during which ¹⁷O water containing at least ¹⁷O having a natural abundance ratio is produced in the subject by oxygen metabolism of the administered ¹⁷O gas.

In the present embodiment, an MRS sequence is used as the imaging sequence in order to obtain spatial position information of the ¹⁷O nucleus detected from the subject. The MRS sequence is provided for obtaining the spatial position information in a subject by phase encoding. Usually, 1 to 3 are selected as the number of encodings. In the present embodiment, the number of encodings of the MRS sequence is not particularly limited.

Then, in a state where the subject S has been placed on the table 8, the static magnetic field generation unit 2 is controlled to generate a static magnetic field. The gradient magnetic field generation unit 3 applies a gradient magnetic field pulse required in accordance with the imaging sequence into the static magnetic field. Then, the subject S is irradiated with an RF pulse generated by the radio-frequency pulse generator 4 in accordance with the pulse sequence, from the irradiation detection coil of the NMR probe 5 (Step St2).

In the next Step St3, an NMR signal generated from the ¹⁷O nucleus of the ¹⁷O water in the subject S, which has been excited by the irradiation with the RF pulse, is detected by the irradiation detection coil of the NMR probe 5. The detected NMR signal is amplified in the signal receiving system 6, phase-detected, and then divided into signals in the two orthogonal systems. The two orthogonal signals are digitally converted, and transmitted to the computer 7. In the computer 7, the transmitted signal is processed by the signal processing unit 15 in accordance with the imaging sequence to generate an NMR spectrum of ¹⁷O. The NMR signal received from the receiving system 6 and the generated NMR spectrum are stored in the storage unit 16, and are displayed on a screen as an image or output by printing or the like, by the output device 18 if necessary.

### Examples

In the present example, Varian NMR System 400WB manufactured by Varian Associated Inc. (Palo Alto, CA, U.S.A.), which is a multi-nuclear NMR apparatus having high spatial resolution and being capable of micro-imaging measurement, was used as the NMR apparatus 1. A saddle-type probe coil (diameter 32 mm x width 40 mm) manufactured by Valtec Co., Ltd. (Joto-ku, Osaka-shi, Osaka, Japan) was used for the static magnetic field generation unit 2. A predetermined pulse sequence shown in FIG. 3 was used as the measurement pulse sequence. The predetermined pulse sequence was obtained by improving the spuls sequence provided as the standard in the multi-nuclear NMR apparatus. In FIG. 3, the horizontal axis is the time, A is the repetition time TR (= cycle time), Tx is the observation nucleus (= ¹⁷O), pd is the delay time, and hard is the broadband pulse.

(Example 1) 6 types (0.04% by weight, 0.06% by weight, 0.12% by weight, 0.20% by weight, 0.29% by weight, and 0.50% by weight) of pure water having different ¹⁷O concentrations, that is, ¹⁷O water, were prepared as subjects. 5 mL of each type of ¹⁷O water was added to a commercially available 15 mL plastic centrifuge tube to create a phantom as a pseudo-biological model that mimics biological tissue in a magnetic resonance manner. Using the created phantom as a sample tube, the NMR measurement method according to the present embodiment described above was applied to detect ¹⁷O present in each type of ¹⁷O water, and examine the correlation relation between the obtained NMR signal intensity and the ¹⁷O concentration.

### Measurement conditions were set as follows.

< Measurement Conditions>
TR: 50 msec
RF pulse: 40 psec hard pulse
Spectrum measurement width (SW): 100,000 Hz
Number of scans (number of integrations): 128

Irradiation with the excitation pulse was performed in accordance with the predetermined pulse sequence under the above measurement conditions, and an NMR signal of the excited ¹⁷O nucleus was detected. The peak intensity (peak amplitude) of the obtained NMR spectrum was imaged in accordance with the MRS sequence. Table 1 as follows shows the peak intensity of the NMR signal obtained for each type of ¹⁷O water as the signal intensity with respect to the ¹⁷O concentration.

**[Table 1]**

| ¹⁷O Concentration (% by weight) | Peak intensity |
|---|---|
| 0.04 | 60.94 |
| 0.06 | 89.51 |
| 0.09 | 134.43 |
| 0.14 | 299.50 |
| 0.29 | 434.57 |
| 0.50 | 862.47 |

As can be understood from Table 1, even though the ¹⁷O concentration is as low as 0.04 which is close to 0.038% of the natural abundance ratio, the peak intensity (signal intensity) is 60.94, and thus it is possible to measure the ¹⁷O nucleus of ¹⁷O water with high sensitivity. This value indicates that the sensitivity in one cycle time is improved by 100 times or more as compared with the measurement by a general pulse sequence for measuring ¹H nuclei in ¹⁷O water.

The experiment results show that, by significantly reducing the cycle time, it is possible to perform measurements that can be integrated several tens of times, during one cycle time of the general pulse sequence, and thereby it is possible to improve the sensitivity by nearly 500 times. It is understood that, according to the NMR measurement method of the present invention, since the cycle time of the imaging sequence (MRS sequence) is set to be very short corresponding to the relaxation time of the ¹⁷O nucleus, ¹⁷O nuclei may be measured with high sensitivity and a high speed even when ¹⁷O water having the ¹⁷O concentration, which is higher than the natural abundance ratio, is not provided.

### (Comparative Example 1)

An experiment of performing irradiation with an RF pulse in accordance with the conventional pulse sequence generally used in the NMR measurement performed by exciting ¹³C nuclei, and detecting an NMR signal generated from ¹⁷O nuclei present in 1% ¹⁷O water (H₂¹⁷O) being a subject was performed. The acquisition time of the pulse sequence was set to 1.5 s, the delay time d1 was set to 689 msec, and the measurement time was 36 s. The static magnetic field strength applied to the subject was set to 1.5 T. The number of scans was 16, the spectrum measurement width was 10,000 Hz, and the number of pieces of data was 15,073.

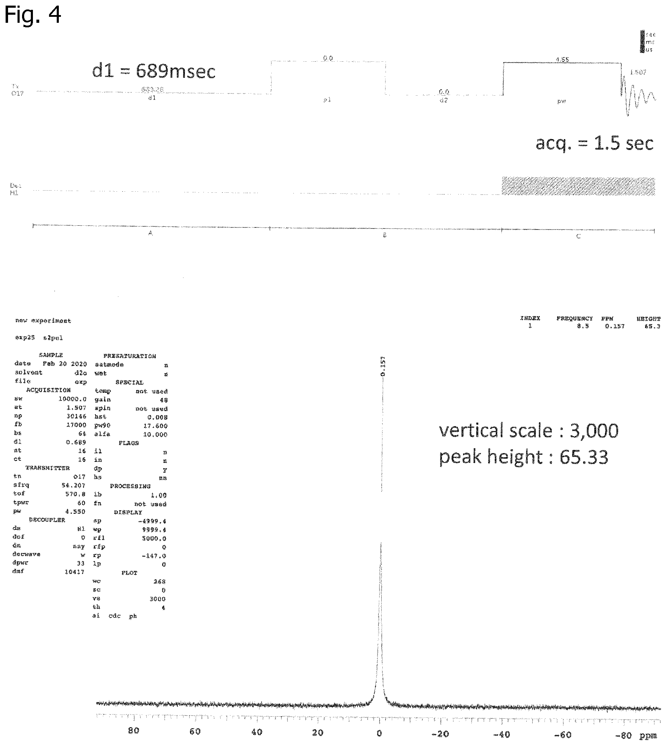

The result is shown in the NMR spectrum diagram of FIG. 4. The detected peak height of the ¹⁷O nuclei was 65.33 in a vertical scale of 3000. This indicates that the peak width of the detected signal is greatly widened, and thus the peak intensity is reduced.

### (Comparative Example 2)

An experiment of performing irradiation with an RF pulse by significantly reducing the acquisition time of the pulse sequence as compared with that of the comparative example 1, that is, setting the acquisition time to 150 msec, which is 1/10 of the acquisition time of the comparative example 1, and detecting an NMR signal generated from ¹⁷O nuclei present in 1% ¹⁷O water (H₂¹⁷O) of the subject was performed. The delay time d1 was set to 689 msec, which is the same as in the comparative example 1, and the measurement time was 13.4 s. The static magnetic field strength applied to the subject was also 1.5 T. The number of scans was 16, the spectrum measurement width was 100,000 Hz, and the number of pieces of data was 15,073.

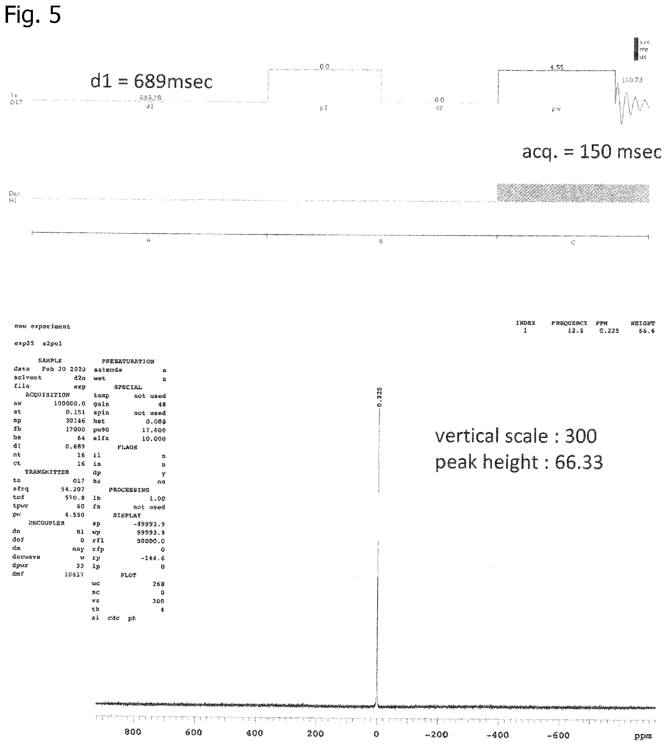

The result is shown in the NMR spectrum diagram of FIG. 5. The detected peak height of the ¹⁷O nuclei was 66.33 in a vertical scale of 300. The sensitivity was improved by about 10 times, the time sensitivity was improved by 2.7 times, and the absolute sensitivity was improved by 27 times, as compared with that of the comparative example 1, but it was not possible to measure the ¹⁷O nucleus having the natural abundance ratio with high sensitivity.

### (Comparative Example 3)

An experiment of performing irradiation with an RF pulse by reducing the delay time d1 of the pulse sequence as compared with in the comparative example 2, that is, setting the delay time d1 to 100 msec, which is a fraction of the delay time of the comparative example 1, and detecting an NMR signal generated from ¹⁷O nuclei present in 1% ¹⁷O water (H₂¹⁷O) of the subject was performed. The acquisition time was set to 150 msec, which is the same as in the comparative example 2, and the measurement time was only 4 s. The static magnetic field strength applied to the subject was also 1.5 T. Similar to the comparative example 2, the number of scans was 16, the spectrum measurement width was 100,000 Hz, and the number of pieces of data was 15,073.

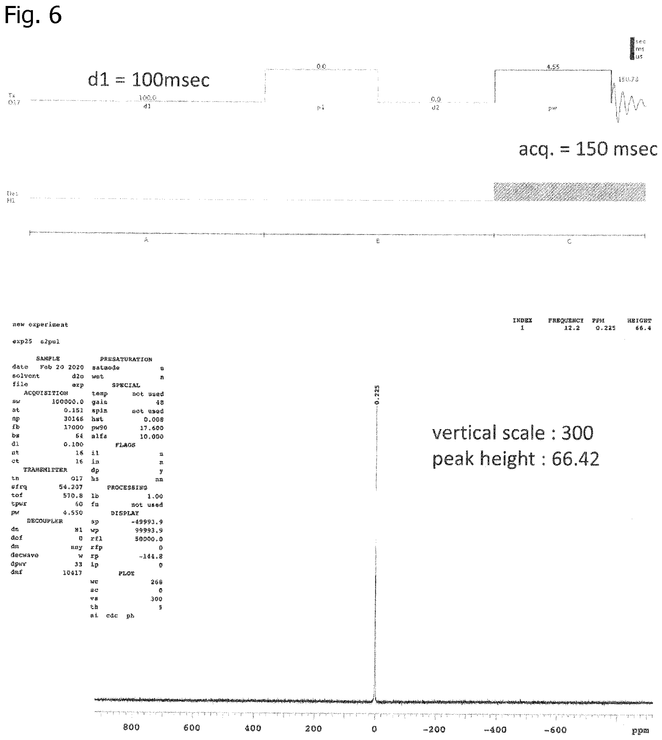

The result is shown in the NMR spectrum diagram of FIG. 6. The detected peak height of the ¹⁷O nuclei was 66.42 in a vertical scale of 300. The cycle time was shortened to about 30% of the cycle time in the comparative example 2, but the sensitivity was improved only about 10 times as compared with the comparative example 1, so as to be equal to the sensitivity in the comparative example 2. The time sensitivity was improved by 9 times, and the absolute sensitivity was improved by 90 times, but it was not possible to measure the ¹⁷O nucleus having the natural abundance ratio with high sensitivity.

Compared with the results of the comparative examples 1 to 3, in the example 1 described above, the sensitivity of the NMR measurement was improved to the extent that ¹⁷O nuclei having a natural abundance ratio could be measured. Thus, it was confirmed that, according to the method of the present invention, since the cycle time of the pulse sequence is set to be very short corresponding to the relaxation time of the ¹⁷O nucleus, the ¹⁷O nuclei may be measured with high sensitivity and a high speed.

### (Example 2)

Using 6 types (0.04% by weight, 0.06% by weight, 0.12% by weight, 0.20% by weight, 0.29% by weight, and 0.50% by weight) of pure water having different ¹⁷O concentrations, that is, ¹⁷O water, as subjects, as in the example 1, the signal intensity of an NMR signal of a ¹⁷O nucleus, which was obtained when proton decoupling of eliminating the influence of scalar coupling between the ¹H nucleus and the ¹⁷O nucleus was performed, was measured. Each type of ¹⁷O water was injected into a commercially available 15 mL plastic centrifuge tube to form a phantom, and ¹⁷O was detected, using the phantom as a sample tube, by the NMR measurement method according to the present embodiment described above.

The measurement time (data taking time) of the ¹⁷O nucleus was set to 104 seconds. Other measurement conditions are the same as in the example 1.

Proton decoupling was performed by irradiating the ¹H nucleus with an RF pulse during the data taking time for the ¹⁷O nucleus. The irradiation with the RF pulse was performed by using a Waltz 16 pulse sequence with a ¹H nuclear signal (399.874 MHz) of ¹⁷O water as a center frequency, a width of ±5,000 Hz, and a relative voltage amount of 47 dB. The irradiation time for the RF pulse may be an irradiation (complete irradiation) time in which the scalar coupling between the ¹H nucleus and the ¹⁷O nucleus can be eliminated, and may not be the same as the measurement time for the ¹⁷O nucleus.

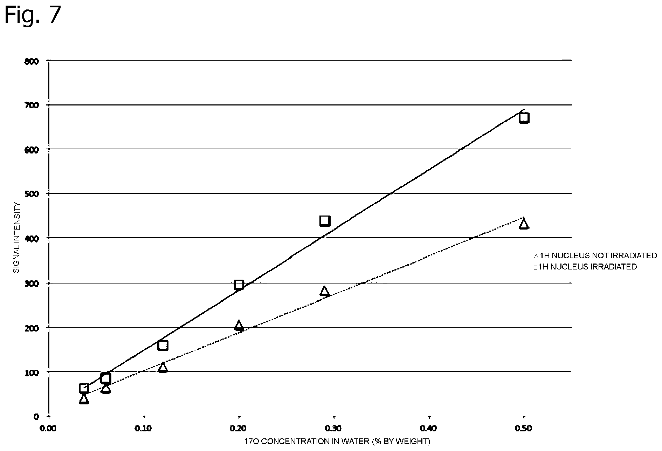

In order to confirm the effect of proton decoupling, the signal intensity of the NMR signal of the ¹⁷O nucleus obtained when the proton decoupling was not performed, that is, when the irradiation with the RF pulse was not performed during the data taking time for the ¹⁷O nucleus was also measured. The measurement results are shown in FIG. 7.

From FIG. 7, it was understood that the signal intensity of the ¹⁷O nucleus has linearity with respect to the ¹⁷O concentration regardless of the presence or absence of proton decoupling. Furthermore, it was confirmed that, comparing the presence and absence of proton decoupling with each other, the signal intensity of the ¹⁷O nucleus when proton decoupling was performed was higher than the signal intensity when proton decoupling was not performed.

In the example 2, by reducing the cycle time and performing proton decoupling as described above, the signal intensity in one cycle time is improved by 100 times or more, and the time sensitivity (obtained by multiplying the number of measurements per unit time and the signal intensity) is improved by about 500 times, as compared with the normal ¹⁷O-MRS measurement. From the experimental result, it can be understood that, according to the NMR measurement method of the present invention, since the cycle time of the imaging sequence (MRS sequence) is set to be very short corresponding to the relaxation time of the ¹⁷O nucleus, and further proton decoupling is performed, the ¹⁷O nuclei may be measured with high sensitivity and a high speed even when ¹⁷O water having the natural abundance ratio is provided.

### (Example 3)

An in-vivo experiment of measuring ¹⁷O water having a natural abundance ratio of 0.038% in a mouse brain by applying the method according to the present embodiment described above was performed. The mouse was subjected to intraperitoneal anesthesia and fixed to a saddle-type probe coil (diameter of 32 mm x width of 40 mm) manufactured by Valtec Co., Ltd., and a ¹⁷O signal was measured by the above-described NMR measurement method according to the present invention.

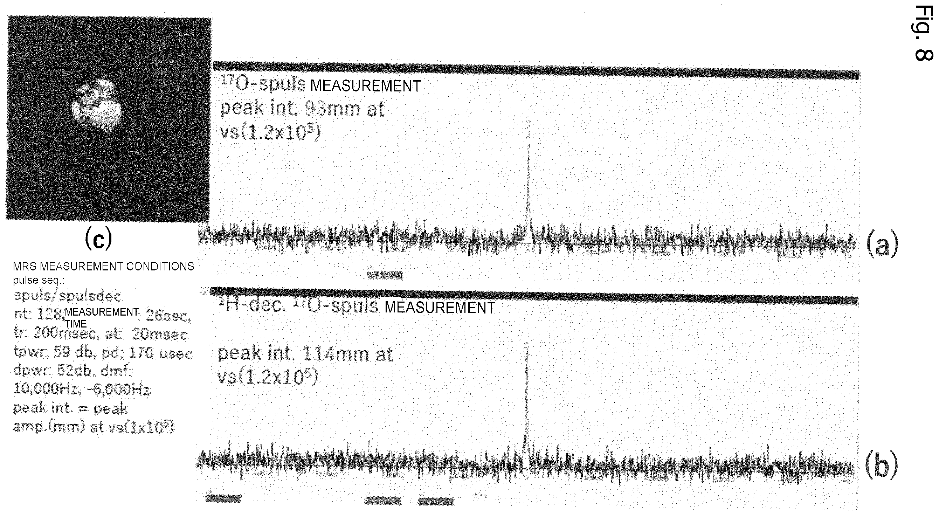

MRS measurement conditions are as follows. In a spuls sequence, the cycle time was set to 200 msec, the acquisition time was set to 20 msec, the number of scans was 128, and the measurement time was 26 s. illustrate NMR spectra obtained as a result is shown in FIGs. 8(a) and 8(b). FIG. 8(c) shows a ¹H tomographic image of the mouse brain illustrating the measurement position.

FIG. 8(a) is an NMR spectrum diagram showing the result of ¹⁷O nuclei detection when proton decoupling has not been performed. As shown in FIG. 8(a), the measured signal intensity of the ¹⁷O nuclei was 93 mm. From this result, it was confirmed that the ¹⁷O nucleus of ¹⁷O water, which is produced by oxygen metabolism of a ¹⁷O gas and is present in a subject at the natural abundance ratio, particularly, being a living body (animal), may be measured with high sensitivity.

FIG. 8(b) is an NMR spectrum diagram showing the result of ¹⁷O nuclei detection when proton decoupling has been performed. As shown in FIG. 8(b), the measured signal intensity of the ¹⁷O nuclei was 114 mm. From this result, it was confirmed that, by performing proton decoupling, the ¹⁷O nucleus of ¹⁷O water, which is produced by oxygen metabolism of a ¹⁷O gas in the living body and is present in the natural abundance ratio may be measured with higher sensitivity.

### (Example 4)

In order to examine the cycle time (TR) in accordance with the relaxation time, which is optimal for measuring ¹⁷O-NMR in a living body, ¹⁷O water having the natural abundance ratio in a mouse brain that is 0.038% was observed as a ¹⁷O signal by the above-described NMR measurement method according to the present invention. The mouse was subjected to intraperitoneal anesthesia and fixed to a ¹⁷O/¹H double resonance surface coil manufactured by Takashima Seisakusho Co., Ltd. (Itabashi-ku, Tokyo, Japan), and the measurement using the following spuls measurement conditions was performed.

As described above in relation to the prior art, the measurement conditions were set as follows in consideration that, for a ¹⁷O nucleus having a quadrupole bipolar moment, the relaxation time is short because the half-value width of an NMR signal depends on the electronic state around the ¹⁷O molecule, and, for a bonded proton atom of a water molecule, the relaxation time of ¹⁷O is extremely short, and that the cycle time optimal for ¹⁷O-NMR is reported to be 15 msec.

### < Measurement Conditions>

SW: 10,000 to 416,666 Hz
Data points: 500 to 5,000
RF pulse: 130 psec hard pulse
TR: 2.8 msec to 200.4 msec
Number of scans: 8,000 or 1,000

The cycle time TR of the spuls sequence used for the ¹⁷O-NMR measurement in the mouse brain was changed between 2.8 msec and 200.4 msec, and the change in the detected ¹⁷O nuclear NMR signal (¹⁷O signal) was examined. Table 2 as follows shows the signal intensity (peak intensity) of the obtained ¹⁷O signal.

**[Table 2]**

| TR (msec) | Signal intensity (mm) | S/N ratio |
|---|---|---|
| 2.8 | 347.7 | 93.19 |
| 5.6 | 215.2 | 27.23 |
| 10.4 | 136.6 | 13.81 |
| 20.4 | 67.4 | 11.49 |
| 50.8 | 27.6 | 6.48 |
| 100.4 | 16.8 | 4.56 |
| 200.4 | - | 5.84 |

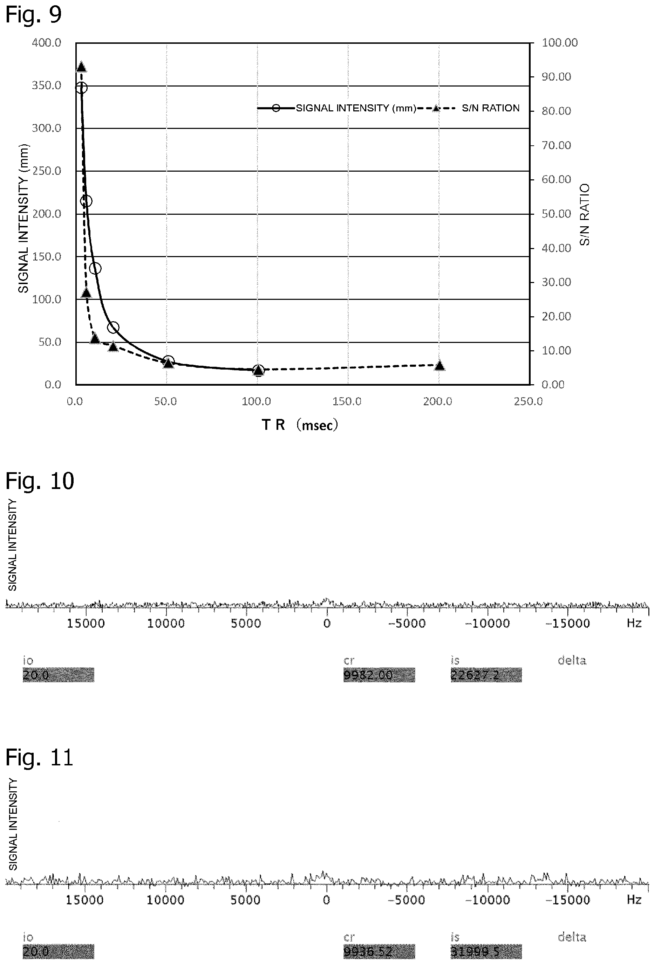

FIG. 9 is a graph showing the measurement results in Table 2 with the horizontal axis of the cycle time (TR) and the vertical axes of the signal intensity and the S/N ratio. From FIG. 9, it was understood that, in chemical shift imaging of ¹⁷O-NMR in the living body, the signal intensity and the S/N ratio of the ¹⁷O signal change substantially in a reciprocal relationship with respect to the cycle time. Specifically, the signal intensity gradually increases as the cycle time changes from 100.4 msec to 20.4 msec, and the rate of increase increases when the cycle time is shorter than 20.4 msec. The signal intensity increases sharply, particularly, when the cycle time is shorter than 10.4 msec. This tendency is similar for the S/N ratio. The S/N ratio shows the tendency as follows. The S/N ratio is almost flat when the cycle time is from 200.4 msec to 50.8 msec. The S/N ratio starts to gradually increase when the cycle time is shorter than 50.8 msec. When the cycle time is equal to or shorter than 20.4 msec, the rate of increase of the S/N ratio increases. In particular, when the cycle time is equal to or shorter than 10.4 msec, the S/N ratio increases sharply. That is, it was clarified that the detection sensitivity of ¹⁷O nuclei starts to be improved at TR = 20.4 msec or shorter, and is improved sharply particularly at TR = 10.4 msec or shorter.

In light of the signal intensity of the result shown in FIG. 9, it is considered that the detection limit of the ¹⁷O signal is the cycle time = 100 msec. Further, considering the S/N ratio, it can be said that the cycle time that can be used for ¹⁷O-NMR measurement in the subject is equal to or shorter than 20.4 msec in the detectable range of 100 msec or shorter. Particularly, in the sense that ¹⁷O nuclei present in the living body at the natural abundance ratio or at the same level concentration, and further ¹⁷O nuclei of ¹⁷O water produced by oxygen metabolism of a ¹⁷O gas in the living body are measured with high sensitivity, it was clarified that the more preferred cycle time is further shorter than 15 msec reported in NPTL3 described above and is equal to or shorter than 10.4 msec.

In the example 4, due to the specifications of the used NMR apparatus (Varian NMR System 400WB), it was not possible to set the cycle time to be shorter than 2.8 msec and perform ¹⁷O-NMR measurement. However, FIG. 9 clearly shows that, if the cycle time is shorter than 2.8 msec in the ¹⁷O-NMR measurement of the example 4, the sensitivity of the detected ¹⁷O signal tends to be further improved.

### (Example 5)

By using the above-described NMR measurement method according to the present invention, ¹⁷O water having a natural abundance ratio of 0.038% in a mouse brain was observed as a ¹⁷O signal without administration of oxygen isotope. Similar to the example 3, a saddle-type probe coil manufactured by Valtec Co., Ltd. was used as the NMR probe 5, and the mouse was subjected to intraperitoneal anesthesia and fixed to a ¹⁷O/¹H double resonance surface coil manufactured by Takashima Seisakusho Co., Ltd. Then, a ¹⁷O signal was measured by using spuls measurement conditions under ¹H decoupling conditions. The number of integrations was set to 1,000, and the measurement was performed when TR was in 4 steps of 20.4, 10.4, 5.6, and 2.8 msec. Table 3 as follows shows the results.

**[Table 3]**

| TR (msec) | Signal intensity | S/N ratio |
|---|---|---|
| 20.4 | 3.70 | 4.20 |
| 10.4 | 4.34 | 2.91 |
| 5.6 | 20.75 | 5.40 |
| 2.8 | 28.17 | 6.58 |

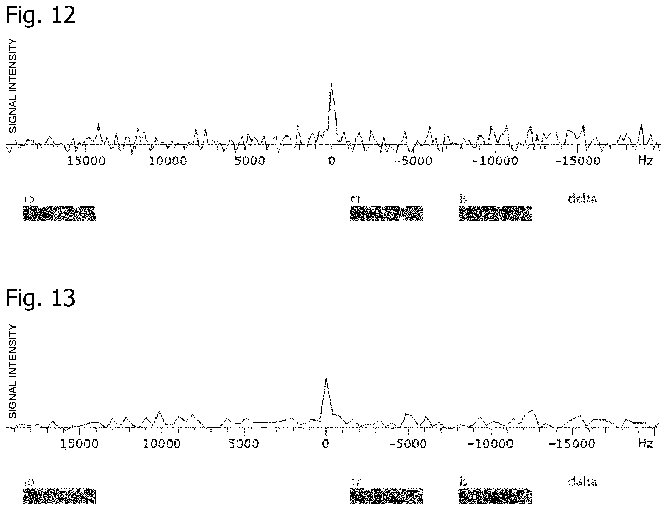

FIGs. 10 to 13 are NMR spectrum diagrams showing the detection results for ¹⁷O nuclei obtained respectively for TR = 20.4, 10.4, 5.6, and 2.8 msec. As shown in Table 3, the signal intensities in the case of TR = 5.6 and 2.8 msec are sufficiently superior to the S/N ratio, respectively. Furthermore, it is clear that the ¹⁷O signal may be detected with high sensitivity from the signal intensities of the ¹⁷O nuclei shown in FIGs. 13 and 12.

On the other hand, the signal intensities in the case of TR = 20.4 and 10.4 msec are about the same as the S/N ratio, respectively. Considering the results, it was confirmed that the cycle time effective for measuring the ¹⁷O nucleus of ¹⁷O water present in the living body at the natural abundance ratio, with high sensitivity, is equal to or shorter than 10.4 msec in a time range shorter than 20.4 msec, and, particularly 5.6 msec or shorter is preferable.

### Description of Reference Numerals

- 1: NMR apparatus
- 2: static magnetic field generation unit
- 3: gradient magnetic field generation unit
- 4: radio-frequency pulse generation unit
- 5: probe
- 6: signal receiving system
- 7: computer
- 14: control unit
- 15: signal processing unit
- 17: input unit

## Claims

1. A nuclear magnetic resonance measurement method comprising steps of:
placing a subject in a predetermined uniform static magnetic field and irradiating the subject with an excitation pulse generated in accordance with a predetermined pulse sequence;
detecting an NMR signal generated by exiting a ¹⁷O nucleus of ¹⁷O water present in the subject at a natural abundance ratio with the excitation pulse; and
processing the detected NMR signal,
wherein a cycle time of the predetermined pulse sequence of the excitation pulse is 20.4 msec or shorter.

2. The nuclear magnetic resonance measurement method according to claim 1, wherein the cycle time of the predetermined pulse sequence is 10.4 msec or shorter.

3. The nuclear magnetic resonance measurement method according to claim 2, wherein the cycle time of the predetermined pulse sequence is 5.6 msec or shorter.

4. The nuclear magnetic resonance measurement method according to claim 3, wherein the cycle time of the predetermined pulse sequence is in a range of 5.6 msec to 2.8 msec.

5. The nuclear magnetic resonance measurement method according to any one of claims 1 to 4, wherein the irradiation with the excitation pulse is performed under proton decoupling.

6. The nuclear magnetic resonance measurement method according to any one of claims 1 to 5, further comprising a step of administering a ¹⁷O gas to the subject, which is a living body, before placing the subject in the static magnetic field,
wherein the NMR signal generated by exiting the ¹⁷O nucleus of ¹⁷O water produced in the living body by oxygen metabolism of the ¹⁷O gas is excited with the excitation pulse is detected.

7. The nuclear magnetic resonance measurement method according to any one of claims 1 to 6, further comprising a step of applying a gradient magnetic field pulse to the static magnetic field, the gradient magnetic field generated in accordance with a predetermined imaging sequence.

8. A nuclear magnetic resonance apparatus comprising:
a static magnetic field generation unit generating a predetermined uniform static magnetic field in a space where a subject is placed;
a radio-frequency pulse generation unit generating an excitation pulse, which is an RF pulse for exciting a ¹⁷O nucleus present in the subject, in accordance with a predetermined pulse sequence;
an NMR probe irradiating the subject placed in the static magnetic field with the excitation pulse and detecting an NMR signal generated from the ¹⁷O nucleus of the subject excited with the excitation pulse; and
a control unit that controls operations of the static magnetic field generation unit, the radio-frequency pulse generation unit, and the NMR probe,
wherein the control unit controls the radio-frequency pulse generation unit to generate the excitation pulse in accordance with the predetermined pulse sequence having a cycle time of 20.4 msec or shorter.

9. The nuclear magnetic resonance apparatus according to claim 8, wherein the cycle time of the predetermined pulse sequence is set to 10.4 msec or shorter.

10. The nuclear magnetic resonance apparatus according to claim 9, wherein the cycle time of the predetermined pulse sequence is set to 5.6 msec or shorter.

11. The nuclear magnetic resonance apparatus according to claim 10, wherein the cycle time of the predetermined pulse sequence is in a range of 5.6 msec to 2.8 msec.

12. The nuclear magnetic resonance apparatus according to any one of claims 8 to 11, wherein the radio-frequency pulse generation unit generates a decoupling pulse, which is an RF pulse for performing proton decoupling that eliminates an influence of scalar coupling between a ¹H nucleus and a ¹⁷O nucleus of ¹⁷O water, and the NMR probe irradiates the subject with the decoupling pulse.

13. The nuclear magnetic resonance apparatus according to any one of claims 8 to 12, further comprising a gradient magnetic field generation unit applying a gradient magnetic field pulse generated in accordance with a predetermined imaging sequence to the static magnetic field.

14. The nuclear magnetic resonance apparatus according to claim 13, wherein the control unit performs a process for imaging the NMR signal detected by the NMR probe.
